# EUROPEAN PATENT APPLICATION

(11) **EP 1 815 808 A1**
(43) Date of publication of application: **08.08.2007**
(21) Application number: 07002115.9
(22) Date of filing: 31.01.2007
(51) Int. Cl.: A61B 17/28

(54) **Endoscopic treatment tool**

(30) Priority: 01.02.2006 JP 2006025003
(71) Applicant: Fujinon Corporation, Saitama-shi, Saitama (JP)
(72) Inventor: Ohashi, Katsuaki, Saitama-shi Saitama (JP); Akahane, Hidefumi, Saitama-shi Saitama (JP)
(74) Representative: Höhfeld, Jochen

(57) **Abstract**

An endoscopic treatment tool comprises: a first member (13) having a first finger retaining part (16); and a second member (14) having at least one second finger retaining part (17A,17B) in a ring form, wherein one of the first (13) and second members (14) is a support member, and the other one of the first (13) and second members (14) is a slider that is slidably arranged on the support member, and wherein a third finger retaining part (20A,20B) having a recess is provided in a front end of at least one of said at least one second finger retaining part of the second member.
The recess is formed by providing a projection (K) on the front end of at least one of said second finger retaining part.

## Description

### Background of the Invention

### 1. Field of the Invention

The present invention relates to endoscopic treatment tools, and more particularly to the finger retaining structure of a treatment tool, such as a forceps or a high-frequency snare, to be used by being inserted through a tool-passage channel of the endoscope.

### 2. Description of the Related Art

The endoscope is arranged with a CCD (charge coupled device) at the tip of an insertion part, say, of the scope thereof. By taking an image of the body interior to observe, the interior body is observed as a video image on a monitor screen. In the examinations, treatments or operations using such endoscopes, various kinds of treatment tools are used including forceps, high-frequency snares and so on. Namely, the scope is provided with a tool-passage channel. By inserting the treatment tool through the tool-passage channel, the treatment tool at its tip can be introduced from the tip end of the scope into the body interior to observe. By doing so, a foreign matter swallowed or a tissue, etc. for biopsy can be caught and removed, say, by means of the forceps. With a high-frequency snare, excision regions, e.g. polyps, can be captured or so.

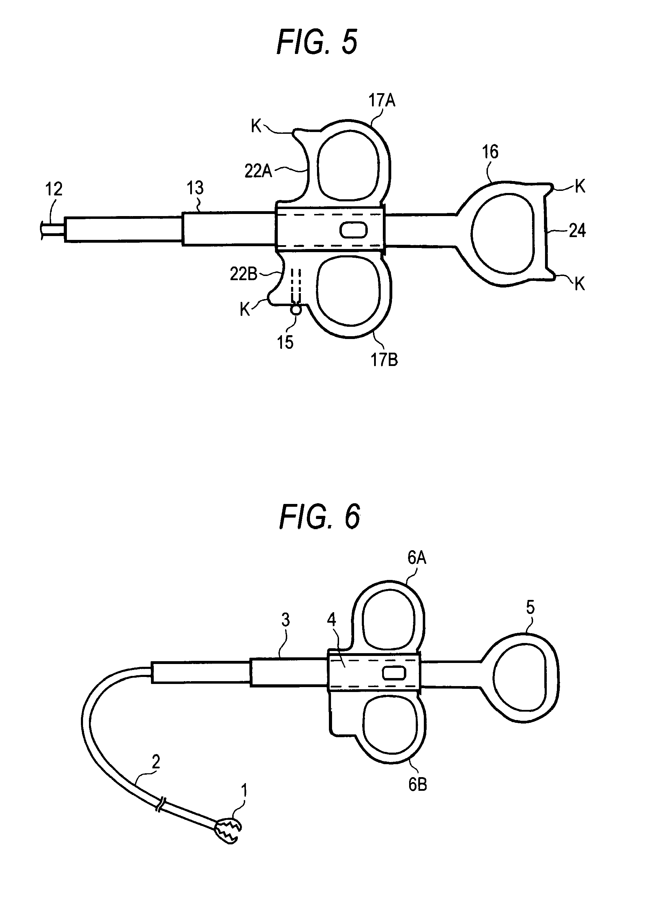

Fig. 6 shows a structure of a forceps serving as a treatment tool of this kind. The forceps is arranged with a linear member 2, having at its tip a grasper 1, arranged up to a hollow tube of a support body 3. A slider 4 is slidably arranged over the outer periphery of the support body 3. The support body 3 has, at its rear end, a ring-formed first finger retaining part 5 to put the thumb thereon. The mating slider 4 has ring-formed second finger retaining parts 6A, 6B provided opposite at the left and right thereof.

With the forceps (treatment tool) thus structured, the silider 4 can be slid longitudinally of the support body 3 by the index and middle fingers put in the second finger retaining parts 6A, 6B while fixing the support body 3 by use of the thumb put in the first finger retaining part 5. As the slider 4 is moved rearward, the grasper 1 closes. As the slider 4 is moved frontward, the grasper 1 opens. By use of the grasper 1, a foreign matter swallowed or a tissue for biopsy can be caught and removed out of the body being observed.

In the meanwhile, the related-art treatment tool (disclosed in e.g. Japanese Patent No. 3,441,640 and JP-A-2005-204998) is structured on the assumption that handling is by one of the hands through use of finger retaining parts 5, 6A, 6B to put the thumb and index and middle fingers therein, as stated above. However, in the treatment, operation or the like using a treatment tool, there is a possibility of requiring a comparatively great force or a constant magnitude of force sustained for a long time in order to operate the slider 4. For example, where to capture and remove a foreign mater, such as a battery cell, swallowed by a children or the like, there is a need to apply a great force and maintain it over a predetermined time. Meanwhile, where to excise polyps by use of a high-frequency snare, a great force is needed upon surrounding and capturing the base portion thereof. In such a case, it is a practice to operate the treatment tool by using the both hands instead of one thereof.

However, in the related-art treatment tool, the finger retaining parts are limited to those for use with one hand. For this reason, the treatment tool is operated by putting the fingers of the other hand on the front ends of the second finger retaining parts. In such a case, there is a fear of slippage at the finger put on the front end of the second finger-retaining part 6A, 6B. Thus, there encounters problematically a difficulty in stably applying a great force or a sustaining force to the treatment tool.

### Summary of the Invention

The present invention has been made in view of the foregoing problem, and it is an object thereof to provide an endoscopic treatment tool easy to operate even with both of the hands whereby stable operation is available by applying a desired magnitude or force or a sustaining force.

In order to achieve the object, according to a first aspect of the invention, there is provided an endoscopic treatment tool comprising: a first member having a first finger retaining part; and a second member having at least one second finger retaining part in a ring form, wherein one of the first and second members is a support member, and the other one of the first and second members is a slider that is slidably arranged on the support member, and wherein a third finger retaining part having a recess is provided in a front end of at least one of said at least one second finger retaining part of the second member. Incidentally, the first finger retaining part may be made in a ring form, or in an arcuate or rod form.

According to a second aspect of the invention, the front end of at least one of said at least one second finger is linearly formed, and a projection is provided at the front end of a least one of said at least one second finger, so as to form the recess (concave face) of the third finger retaining part.

According to a third aspect of the invention, the first finger retaining part is formed in a ring form, so as to form the recess of a fourth finger retaining part in a rear end of the first finger retaining part.

According to the above structure, the slider can be moved relatively to the support body of the treatment tool, say, by putting the thumb of the right hand on the first finger retaining part and the index and middle fingers thereof in the ring-formed second finger retaining parts while putting the thumb of the left hand on the first finger retaining part (or the fourth finger retaining part) and the index and middle fingers thereof on the third finger retaining parts in the front end of the second finger retaining parts.

### Brief Description of the Drawings

Fig. 1 is a perspective view showing a structure of an endoscopic treatment tool (treatment component) according to a first embodiment of the present invention;
Fig. 2 is a front view showing a structure of a treatment tool in the first embodiment;
Fig. 3 is a perspective view showing a state that the treatment tool of the first embodiment is operated with one hand;
Fig. 4 is a perspective view showing a state that the treatment tool of the first embodiment is operated with both hands;
Fig. 5 is a front view showing a structure of a treatment tool in a second embodiment; and
Fig. 6 is a front view showing a structure of a related-art treatment tool (forceps).

### Detailed Description of the Invention

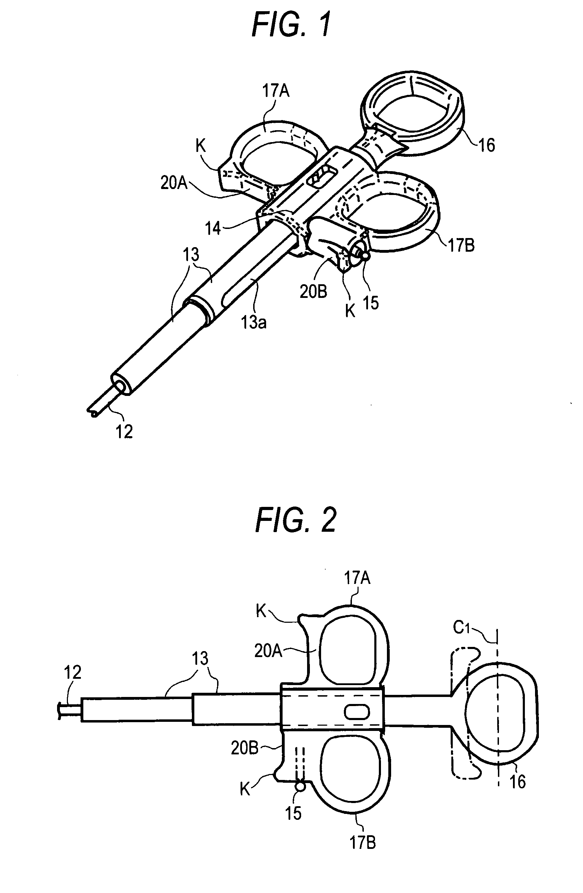

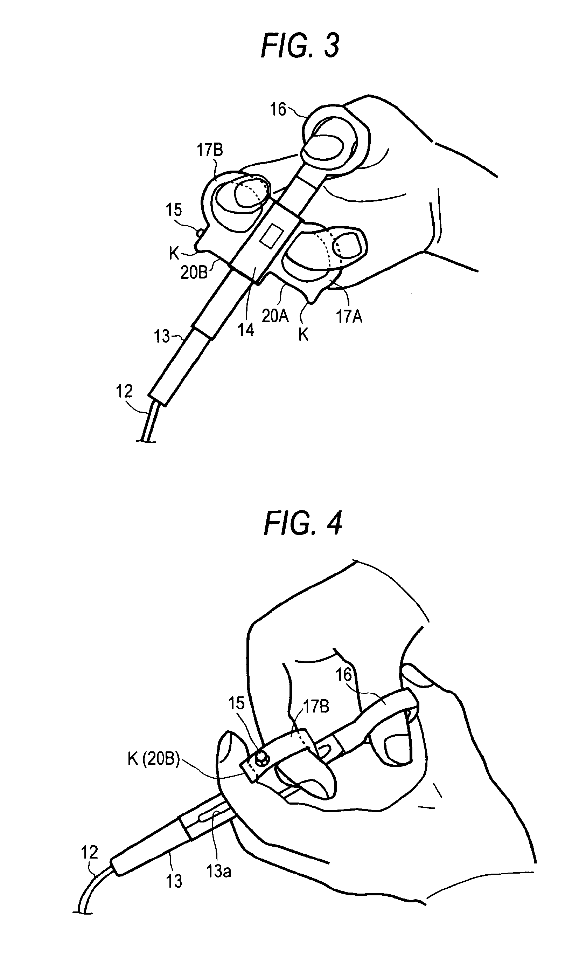

Figs. 1 and 2 show a structure of an endoscopic treatment tool (operation component) according to a first embodiment. Figs. 3 and 4 show the operation status of the treatment tool in the embodiment. The treatment tool may be a bioptome, a collection forceps, a high-frequency snare, a high-frequency hemostatic tool, diathermy knife or the like but, in the embodiment, shows a tool making use of high frequency.

As shown in Fig. 1, in the treatment tool, a linear member 12 having at its tip a manipulator, e.g. catcher, is arranged in a support body (support fixing portion) 13 (hollow tube) serving as a first member. The support body 13 is slidably fit with a slider 14 serving as a second member, through a guide hole 13a. In the embodiment, a high-frequency connector 15 is arranged in the slider 14 in a position close to the tip thereof. The support body 13 is provided with a first finger retaining part 16 in a ring form (hoop, circular or elliptic in form) to put therein the thumb of the right (or left) of the hands. Instead of the ring form, the first finger retaining part 16 may be formed as a semicircular finger retaining part which the ring-formed first finger retaining part 16 is cut of its rear half along the cut line C₁ as shown in Fig. 2 or as a finger retaining part that such a semicircular form is spread into a linear form (T-form as shown by the two-dot chain line).

Meanwhile, the slider 14 is provided with second finger retaining parts 17A, 17B to put therein the index and middle fingers of the right hand, at its both left and right sides thereof. The second finger retaining parts 17A, 17B are respectively provided with third finger retaining parts 20A, 20B to put thereon the index and middle fingers of the other, i.e. left, (or the right hand when the left hand is put in the second finger retaining parts 17A, 17B) of the hands, at the respective front ends thereof. Namely, as shown in Fig. 2, by providing a projection K in the outer position of a linear (or flat) front face as viewed from front of the ring's front end of the second finger retaining part 17A, the third finger retaining part 20A is formed with a recess (recessed surface). By providing a projection K in the outer position of a linear front face of the ring's front end (in front of the high-frequency connector 15) of the second finger retaining part 17B, the third finger retaining part 20B is formed serving as a recess (recessed surface).

According to the treatment tool of the first embodiment thus structured, when operated only with one hand, the thumb of the right hand, for example, is put in the first finger retaining part 16 while the index and middle fingers thereof are respectively put in the second finger retaining part 17B and the second finger retaining part 17A. By moving the thumb and the index and middle fingers, the slider 14 is moved lengthwise toward the rear (rearward) of the support body 13. In the case of a high-frequency snare, the hoop-formed manipulator can be closed at its hoop. In the case of a forceps, its catcher can be closed.

Meanwhile, where manipulated with the both hands, the thumb of the left hand is put on the rear end (outer side) of the first finger retaining part 16 as shown in Fig. 4 while the index and middles fingers thereof are put respectively on the third finger retaining part 20B and the third finger retaining part 20A, in addition to the arrangement of the fingers of the right hand. With the both hands, the slider 14 can be moved back and forth lengthwise of the support body 13. By such operation with both hands, the greater force can be applied to the treatment tool as compared to that with one hand. Furthermore, the force can be sustained longer in time.

Fig. 5 shows a structure according to a second embodiment. In the second embodiment, the third finger retaining parts 22A, 22B are formed as arcuate recesses, as shown in the figure. Namely, the second finger retaining parts 17A, 17B respectively have front surfaces formed arcuate as viewed from front with the result that recesses are respectively formed with projections K in the outer positions thereof. Meanwhile, in the rear face of the first finger retaining part 16, a forth finger retaining part 24 is provided with projections K at its left and right so that the thumb of the left hand can be put thereon.

According to the second embodiment, the treatment tool is facilitated to operate with the both hands, say, by putting the index and middle fingers of the left hand respectively on the third finger retaining parts 22A, 22B. By putting the thumb of the left hand on the fourth finger retaining part 24, stable treatment is realized while applying the greater force or the sustaining force for a predetermined time.

Although the embodiments explained that the first member having the first finger retaining part was taken as the support body 13 while the second member having the second finger retaining part is as the slider 14, the second member may be structured as a support body while the first member is as a slider. Meanwhile, the third finger retaining part (20A, 20B, 22A, 22B) may be arranged in any one of the second finger retaining parts 17A, 17B.

According to the endoscopic treatment tool of the invention, the treatment tool can be easily operated even with both of the hands without limited to one of the hands whereby stable operation is available by applying a desired magnitude of force or a sustaining force. This provides an effect that a foreign matter or a tissue can be removed by means of a forceps or polyps can be well treated, operated or so by means of a high-frequency snare.

The entire disclosure of each and every foreign patent application from which the benefit of foreign priority has been claimed in the present application is incorporated herein by reference, as if fully set forth.

## Claims

1. An endoscopic treatment tool comprising:
a first member having a first finger retaining part; and
a second member having at least one second finger retaining part in a ring form,
wherein one of the first and second members is a support member, and the other one of the first and second members is a slider that is slidably arranged on the support member, and
wherein a third finger retaining part having a recess is provided in a front end of at least one of said at least one second finger retaining part of the second member.

2. An endoscopic treatment tool according to claim 1,
wherein the front end of at least one of said at least one second finger is linearly formed, and
a projection is provided at the front end of at least one of said at least one second finger, so as to form the recess of the third finger retaining part.

3. An endoscopic treatment tool according to claim 1,
wherein the first finger retaining part is formed in a ring form, so as to form the recess of a fourth finger retaining part in a rear end of the first finger retaining part.
